(19) Europäisches Patentamt
European Patent Office
Office européen des brevets

(11) Numéro de publication : 0 333 527 B1

(12) # FASCICULE DE BREVET EUROPEEN

(45) Date de publication du fascicule du brevet : 27.11.91 Bulletin 91/48

(51) Int. Cl.[5] : C07C 319/06

(21) Numéro de dépôt : 89400330.0

(22) Date de dépôt : 06.02.89

(54) **Procédé de préparation de dithiols.**

(30) Priorité : 17.02.88 FR 8801881

(43) Date de publication de la demande : 20.09.89 Bulletin 89/38

(45) Mention de la délivrance du brevet : 27.11.91 Bulletin 91/48

(84) Etats contractants désignés : AT BE CH DE ES FR GB GR IT LI LU NL SE

(56) Documents cités :
EP-A- 0 047 021
EP-A- 0 082 500
US-A- 3 069 472

(73) Titulaire : SOCIETE NATIONALE ELF AQUITAINE (PRODUCTION)
Tour Elf, 2, Place de la Coupole, La Défense 6
F-92400 Courbevoie (FR)

(72) Inventeur : Arretz, Emmanuel
2 Rue de Cagnes
F-64000 Pau (FR)

(74) Mandataire : Leboulenger, Jean et al
ATOCHEM Département Propriété Industrielle
Cédex 42- La Défense 10
F-92091 Paris la Défense (FR)

EP 0 333 527 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

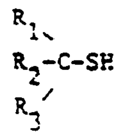

(II)

formed as a byproduct in the reaction is reemployed cyclically for the preparation of the starting di(tert-alkylthio)alkane.

8. Process according to Claim 7, in which the tertiary mercaptan (II) is chosen from those whose boiling point is sufficiently different from that of the final dithiol to enable them to be separated by simple distillation.

9. Process according to Claim 7 or 8, in which the tertiary mercaptan (II) is added photochemically to an unconjugated diene containing at least 5 carbon atoms.

10. Process according to Claim 9, in which from 2 to 4 moles of tertiary mercaptan are used per mole of diene.

11. Process according to Claim 9 or 10, in which the photochemical addition is performed at a temperature ranging from −10°C to +60°C, preferably between 20 and 50°C.

12. Process according to one of Claims 9 to 11, in which the photochemical addition is performed in the presence of a catalyst chosen from acetophenone derivatives, benzoylphosphine oxides and combinations of an aromatic ketone with an organic phosphite or phosphine.

13. Process according to one of Claims 9 to 12, in which the unconjugated diene is an α,ω-diene containing from 5 to 20 carbon atoms.

14. Process according to one of Claims 1 to 13, in which the operation is carried out continuously.

## Patentansprüche

1. Verfahren zur Herstellung von Dithiolen, dadurch gekennzeichnet, daß man in Gegenwart eines festen sauren Katalysators Schwefelwasserstoff mit einem Di-(tert.-alkylthio)-alkan der allgemeinen Formel

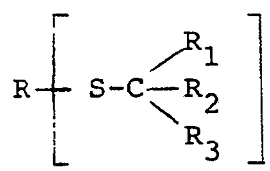

(I)

reagieren läßt, in der R einen 5 bis 20 Kohlenstoffatome enthaltenden Alkylenrest bedeutet und die Symbole $R_1$, $R_2$ und $R_3$ gleich oder verschieden sind und jeweils einen 1 bis 15 Kohlenstoffatome enthaltenden Alkylrest bedeuten.

2. Verfahren nach Anspruch 1, dadurch gekennzeichnet, daß die Umsetzung bei einer Temperatur zwischen Umgebungstemperatur und 250°C und vorzugsweise zwischen 85 und 200°C durchgeführt wird.

3. Verfahren nach Anspruch 1 oder 2, dadurch gekennzeichnet, daß man unter einem Druck von 5 bis 30 bar und vorzugsweise zwischen 10 und 20 bar arbeitet.

4. Verfahren nach einem der Ansprüche 1 bis 3, dadurch gekennzeichnet, daß man 2 bis 12 und vorzugsweise 4 bis 8 mol $H_2S$ pro mol Di-(tert.-alkylthio)-alkan umsetzt.

5. Verfahren nach einem der Ansprüche 1 bis 4, dadurch gekennzeichnet, daß der feste saure Katalysator ein natürliches oder synthetisches Aluminosilicat oder ein Ionenaustauscherharz ist.

6. Verfahren nach einem der Ansprüche 1 bis 5, dadurch gekennzeichnet, daß das Di-(tert.-alkylthio)-alkan ein α,ω-Di-(tert.-alkylthio)-alkan ist.

7. Verfahren nach einem der Ansprüche 1 bis 6, dadurch gekennzeichnet, daß das Reaktionsnebenprodukt, tertiäres Mercaptan der Formel

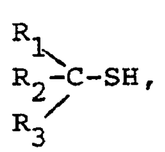

(II)

$$(Mg\text{-}Ca)O \cdot Al_2O_3 \cdot 5SiO_2 \cdot nH_2O$$

commercialisés sous la marque FILTROL.

En ce qui concerne les zéolithes, on préfère celles des types X et Y dont la teneur en sodium, exprimée en $Na_2O$, est inférieure à 15% en poids et avantageusement inférieure à 3%.

Comme résines échangeuses d'ions, on préfère les échangeurs cationiques acides à structure polymérique aromatique (polystyrène et divinylbenzène) ou à structure aliphatique perfluorée, contenant des groupements sulfoniques. Ces résines qui peuvent être utilisées en masse ou fixées sur un support minéral ou organique se trouvent dans le commerce sous les appellations Amberlite, Amberlyst, Levatit, Dowex, Duolite et Nafion.

L'acide phosphorique imprégnant un support peut également convenir comme catalyseur pour la sulfhydrolyse selon l'invention.

Le mercaptan tertiaire (II) est de préférence choisi parmi ceux dont la température d'ébullition est suffisamment différente de celle du dithiol final de façon que leur séparation par distillation soit facile et rende aisé le recyclage du mercaptan tertiaire pour la synthèse du di-(tertioalkylthio)alcane. Comme exemples de mercaptans tertiaires (II), on peut citer plus particulièrement le tertiobutylmercaptan (Eb. 66°C), le tertiooctylmercaptan (Eb. 150°C), le tertiononylmercaptan (Eb. 189-210°C), le tertiododécylmercaptan (Eb. 228-246°C), le tertiohexadécylmercaptan (Eb. 277-316°C).

Les di-(tertioalkylthio)alcanes (I) du procédé selon l'invention peuvent avantageusement être préparés par addition photochimique de deux molécules d'un mercaptan tertiaire de formule (II) sur une molécule d'un diène non conjugué contenant au moins 5 atomes de carbone.

Cette addition photochimique du type Karash peut être réalisée à pression atmosphérique par irradiation, au moyen d'une lampe émettant dans l'ultraviolet (200 à 400 nm), d'un mélange liquide du diène non conjugué et du mercaptan tertiaire (II) en excès, de préférence dans des proportions telles que le rapport molaire mercaptan tertiaire sur diène soit compris entre 2 et 4.

La réaction photochimique peut être réalisée à des températures modérées, généralement comprises entre −10°C et +60°C (de préférence entre 20 et 50°C), et être effectuée en l'absence de catalyseur. Cependant, pour améliorer son rendement et sa productivité, il est préférable d'opérer en présence d'un catalyseur spécifique constitué soit par une cétone aromatique associée à un phosphite ou une phosphine organique, soit par certains dérivés de l'acétophénone, soit encore par un benzoylphosphineoxyde.

Comme cétones aromatiques utilisables, ou peut mentionner la benzophénone et des dérivés de la benzophénone contenant sur l'un ou les deux noyaux benzéniques un ou plusieurs substituants tels que des atomes d'halogène ou des groupements alkyle, alcoxy ou alkylthio, ainsi que les composés xanthéniques de formule :

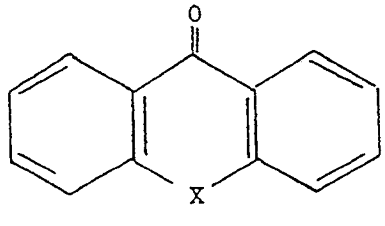

(III)

dans laquelle X désigne un atome d'oxygène ou de soufre et les noyaux benzéniques peuvent porter jusqu'à trois substituants halogène, alkyle ou aryle. Ces cétones sont utilisés en mélange avec un phosphite organique, de préférence un phosphite trialkylique comme le triméthylphosphite, le tributylphosphite, le tridodécylphosphite, ou avec une phosphine organique comme la triméthylphosphine, la triéthylphosphine ou la tributylphosphine.

Comme dérivés de l'acétophénone utilisables à titre de catalyseur, on peut mentionner plus particulièrement les α,α-dialcoxy α-phényl acétophénones, les α-alcoxy α-phényl acétophénones, les α,α-dialcoxy acétophénones, les α,α-dialkyl α-hydroxy acétophénones et les α,α-dialkyl α-morpholino acétophénones dont les groupes alcoxy et alkyle peuvent contenir de 1 à 8 atomes de carbone, ainsi que leurs dérivés substitués sur le ou les noyaux benzéniques, par exemple, par des groupements alkyle, alcoxy, alkylthio.

A titre de catalyseur du type benzoylphosphineoxyde, on peut citer ceux de formule :

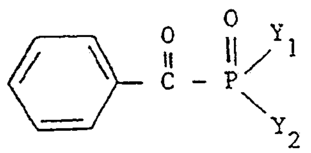

(IV)

dans laquelle le noyau benzénique peut porter divers substituants tels que, par exemple, des groupements alkyle, alcoxy ou alkylthio, et les symboles $Y_1$ et $Y_2$ sont des groupes hydrocarbonés aliphatiques ou aromatiques.

Dans le cas où le système catalytique est un mélange d'un cétone aromatique et d'un phosphite ou d'une phosphine organique, les concentrations à utiliser pour chacun de ces constituants sont de 0,005 à 0,1 mole par litre de diène mis en oeuvre. Pour les autres catalyseurs (acétophénones, benzoylphosphineoxydes), les concentrations à utiliser sont de 0,0001 à 0,1 mole par litre de diène.

Le procédé selon l'invention s'applique en général à la préparation de dithiols à partir de diènes non conjugués comportant de 5 à 20 atomes de carbone. En particulier, des dithiols très utiles, porteurs d'un group SH à chaque extrémité de leur chaîne hydrocarbonée, sont obtenus à partir d'α,ω-diènes. A titre d'exemples non limitatifs de tels α,ω-diènes, on peut citer le pentadiène, l'hexadiène, l'octadiène, le décadiène, l'undécadiène, le dodécadiène, l'hexadécadiène et l'eicosadiène.

Chacune des deux étapes (addition photochimique et sulfhydrolyse) peut être exécutée en discontinu ou en continu. Cependant, dans le mode d'exécution le plus pratique, le procédé est mis en oeuvre en continu et peut fonctionner en procédé cyclique dans lequel le mercaptan tertiaire (II), formé dans l'étape de sulfhydrolyse simultanément avec le dithiol désiré, est recyclé au réacteur photochimique de production du di-(tertioalkylthio)alcane par réaction avec le diène non conjugué.

Les exemples suivants illustrent l'invention sans la limiter.

## EXEMPLE 1 : HEXANEDITHIOL-1,6

a) Préparation du di-(tertiobutylthio)-1,6 hexane

L'addition du tertiobutylmercaptan sur l'hexadiène-1,5 selon la réaction :

$$2(CH_3)_3C\text{-}SH + H_2C\text{=}CH\text{-}(CH_2)_2\text{-}CH\text{=}CH_2 \rightarrow (CH_3)_3C\text{-}S\text{-}(CH_2)_6\text{-}S\text{-}C(CH_3)_3$$

est effectuée dans un réacteur constitué par un cylindre en acier inoxydable, d'un volume utile de 300 ml. Dans l'axe de ce cylindre est fixé un tube coaxial en quartz contenant une lampe à mercure dont le maximum d'émission se situe à la longueur d'onde de 350 nm.

Le refroidissement et l'agitation du milieu liquide sont assurés par une boucle extérieure, comprenant une pompe de recirculation et d'un échangeur permettant de maintenir la température du milieu à 25 ± 2°C.

Dans le réacteur on introduit, par heure, un mélange de 270 g (3 moles) de tertiobutylmercaptan et 82 g (1 mole) d'hexadiène-1,5 contenant 128 mg d'α,α-diméthoxy α-phényl acétophénone (soit environ 0,004 mole par litre de diène). Le débit du liquide sortant du réacteur est mesuré et analysé par chromatographie en phase gazeuse. Le taux de transformation de l'hexadiène-1,5 est de 96%, et la production de di-(tertiobutylthio)-1,6 hexane est de 246 g par heure, ce qui correspond à un rendement de 94% par rapport à l'hexadiène-1,5 mis en oeuvre. On élimine par distillation l'excès de mercaptan et l'hexadiène-1,5 non transformé et les recycle éventuellement en tête du réacteur.

En répétant cette opération, mais avec d'autres catalyseurs, on a obtenu les résultats rassemblés dans le tableau suivant :

| SYSTEME CATALYTIQUE (MOLE/LITRE D'HEXADIENE) | CONVERSION DE L'HEXADIENE (%) | RENDEMENT EN DI-(TERTIOBUTYLTHIO)-1,6 HEXANE (%) |
|---|---|---|
| α,α-Diméthyl α-phényl acétophénone (0,004) | 96 | 94 |
| α-Méthoxy α-phényl acétophénone (0,004) | 88 | 86,1 |
| α-Méthoxy α-phényl acétophénone (0,01) | 92 | 90 |
| α,α-Diméthyl α-hydroxy acétophénone (0,004) | 86,7 | 84,9 |
| α,α-Diéthoxy acétophénone (0,004) | 85 | 83 |
| Benzophénone (0,01) + tributylphosphite (0,02) | 65 | 63,6 |
| Thioxanthone (0,01) + tributylphosphite (0,02) | 62 | 60,7 |

b) Production de l'hexanedithiol-1,6

Pour effecteur la sulfhydrolyse du di-(tertiobutylthio)-1,6 hexane en hexanedithiol-1,6, on utilise un réacteur tubulaire de 25 mm de diamètre, présentant une capacité utile de 200 ml chargée de résine échangeuse de cations, connue sous la dénomination commerciale d'Amberlyst 15, préalablement séchée.

A travers cette charge on fait passer à l'heure 52,4 g (0,2 mole) de di-(tertiobutylthio)-1,6 hexane et 40,8 g (1,2 mole) de $H_2S$.

La pression dans le réacteur est maintenue à 15 bars. La réaction a été étudiée à différentes températures réglées par la circulation d'huile, maintenue à température constante, à travers une double enveloppe qui entoure la réacteur.

Pour chaque essai effectué à une température déterminée, le produit brut de réaction a été analysé. On a ainsi établi le taux de transformation du di-(tertiobutylthio)-1,6 hexane de départ, la production et la sélectivité en hexanedithiol-1,6, ainsi que la sélectivité en produit intermédiairement formé dans la sulfhydrolyse et qui correspond à la conversion d'une des deux fonctions sulfure de di-(tertiobutylthio)-1,6 hexane (composé A) en tertiobutylthio-6 hexanethiol (composé B) qui se transforme par recyclage et augmentation éventuelle de la température de réaction, en le dithiol final.

On peut schématiser ces différentes réactions de la façon suivante :

(1) $(CH_3)_3C\text{-}S(CH_2)_6S\text{-}C(CH_3)_3 + 2H_2S \longrightarrow HS(CH_2)_6SH + 2(CH_3)_3C\text{-}SH$
(A)

(2) $(CH_3)_3C\text{-}S(CH_2)_6S\text{-}C(CH_3)_3 + H_2S \longrightarrow (CH_3)_3C\text{-}S(CH_2)_6SH + (CH_3)_3C\text{-}SH$
(A) (B)

(3) $(CH_3)_3C\text{-}S(CH_2)_6SH + H_2S \longrightarrow HS(CH_2)_6SH + (CH_3)_3C\text{-}SH$
(B)

Les résultats sont donnés dans le tableau suivant :

| TEMPERATURE (°C) | CONVERSION DU COMPOSE A | PRODUCTION D'HEXANEDITHIOL (g/h) | SELECTIVITE | |
|---|---|---|---|---|
| | | | HEXANEDITHIOL | COMPOSE B |
| 110 | 80,2 | 7,3 | 30,5 | 67,1 |
| 130 | 89 | 16 | 60 | 38 |
| 150 | 95 | 21,3 | 75 | 23 |

## EXAMPLE 2

On a répété l'exemple 1-b dans les conditions réactionnelles suivantes :

| | | |
|---|---|---|
| Charge de catalyseur | : | 200 ml |
| Pression opératoire | : | 15 bars |
| Température | : | 150°C |
| Débit horaire en : | | |
| - composé A | : | 62,9 g/h |
| - $H_2S$ | : | 49 g/h |

avec trois catalyseurs différents, à savoir : Amberlyst 15, Filtrol 71 et Zéolithe Y-62 (commercialisé par UNION CARBIDE).

Les résultats obtenus sont présentés dans le tableau suivant :

| CATALYSEUR | CONVERSION DU COMPOSE A | PRODUCTION D'HEXANEDITHIOL (g/h) | SELECTIVITE | |
|---|---|---|---|---|
| | | | HEXANEDITHIOL | COMPOSE B |
| Amberlyst 15 | 91 | 23,6 | 72 | 26 |
| Filtrol 71 | 88 | 22,1 | 70 | 28 |
| Zéolithe Y-62 | 86 | 21,3 | 69 | 29 |

EXEMPLE 3 : DECANEDITHIOL-1,10

a) Préparation du di-(tertiobutylthio)-1,10 décane

On a opéré comme dans l'exemple 1-a, mais en introduisant en continu, par heure, un mélange de 270 g de tertiobutylmercaptan, 138 g de décadiène-1,9 et 179 mg d'α,α-diméthoxy α-phényl acétophénone (soit 0,0038 mole par litre de diène).

Le mélange liquide récupéré à la sortie du réacteur correspond à un taux de transformation du décadiène-1,9 de 91% et à une production de di-(tertiobutylthio)-1,10 décane de 276,7 g par heure, correspondant à un rendement de 87% par rapport au décadiène-1,9 mis en oeuvre. Par distillation, on élimine l'excès de mercaptan et le décadiène-1,9 non transformé.

b) Production du décanedithiol-1,10

On a opéré comme dans l'exemple 1-b avec le réacteur chargé de 200 ml de résine Amberlyst 15, préalablement séchée. A travers cette charge, on a fait passer à l'heure 63,6 g de di-(tertiobutylthio)-1,10 décane et 40,8 g de $H_2S$, la pression dans le réacteur étant maintenue à 15 bars et la température à 140°C.

On a obtenu ainsi 23 g par heure de décanedithiol-1,10, soit un rendement de 56% par rapport au di-(tertiobutylthio)-1,10 décane dont 91% ont été transformés. La transformation complémentaire est essentiellement représentée par le tertiobutylthio-10 décanethiol résultant de la sulfhydrolyse d'une seule fonction sulfure. Ce mercaptansulfure peut être facilement séparé du décanedithiol-1,10 par distillation et recyclé dans une nouvelle opération pour être converti en le dithiol.

## Revendications

1. Procédé de préparation de dithiols, caractérisé en ce que l'on fait réagir, en présence d'un catalyseur acide solide, l'hydrogène sulfuré avec un di-(tertioalkylthio)alcane de formule générale :

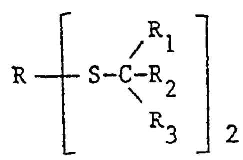

(I)

dans laquelle R représente un radical alkylène contenant de 5 à 20 atomes de carbone, et les symboles $R_1$, $R_2$ et $R_3$, identiques ou différents, représentent chacun un radical alkyle contenant de 1 à 15 atomes de carbone.

2. Procédé selon la revendication 1, dans lequel la réaction est effectuée à une température comprise entre la température ambiante et 250°C, de préférence entre 85 et 200°C.

3. Procédé selon la revendication 1 ou 2, dans lequel on opère sous une pression allant de 5 à 30 bars, de préférence comprise entre 10 et 20 bars.

4. Procédé selon l'une des revendications 1 à 3, dans lequel on met en oeuvre de 2 à 12 moles de $H_2S$,

de préférence de 4 à 8 moles, par mole de di-(tertioalkylthio)alcane.

5. Procédé selon l'une des revendications 1 à 4, dans lequel le catalyseur acide solide est un aluminosilicate naturel ou synthétique ou une résine échangeuse d'ions.

6. Procédé selon l'une des revendications 1 à 5, dans lequel le di-(tertioalkylthio)alcane est un α,ω-di-(tertioalkylthio)alcane.

7. Procédé selon l'une des revendications 1 à 6, dans lequel le mercaptan tertiaire de formule :

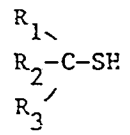

(II)

sous-produit par la réaction est réutilisé cycliquement pour la préparation du di-(tertioalkylthio)alcane de départ.

8. Procédé selon la revendication 7, dans lequel le mercaptan tertiaire (II) est choisi parmi ceux dont la température d'ébullition est suffisamment différente de celle du dithiol final pour permettre leur séparation par simple distillation.

9. Procédé selon la revendication 7 ou 8, dans lequel le mercaptan tertiaire (II) est additionné photochimiquement sur un diène non conjugué contenant au moins 5 atomes de carbone.

10. Procédé selon la revendication 9, dans lequel on met en oeuvre de 2 à 4 moles de mercaptan tertiaire par mole de diène.

11. Procédé selon la revendication 9 ou 10, dans lequel l'addition photochimique est effectuée à une température allant de −10°C à +60°C, de préférence comprise entre 20 et 50°C.

12. Procédé selon l'une des revendications 9 à 11, dans lequel l'addition photochimique est effectuée en présence d'un catalyseur choisi parmi les dérivés de l'acétophénone, les benzoylphosphineoxydes et les combinaisons d'une cétone aromatique avec un phosphite ou une phosphine organique.

13. Procédé selon l'une des revendications 9 à 12, dans lequel le diène non conjugué est un α,ω-diène contenant de 5 à 20 atomes de carbone.

14. Procédé selon l'une des revendications 1 à 13, dans lequel on opère en continu.

## Claims

1. Process for the preparation of dithiols, characterised in that hydrogen sulphide is reacted in the presence of a solid acidic catalyst with a di(tert-alkylthio)alkane of general formula :

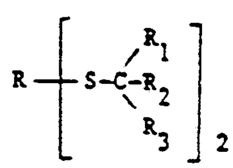

(I)

in which R denotes an alkylene radical containing from 5 to 20 carbon atoms, and each of the symbols $R_1$, $R_2$ and $R_3$, which are identical or different, denotes an alkyl radical containing from 1 to 15 carbon atoms.

2. Process according to Claim 1, in which the reaction is performed at a temperature between room temperature and 250°C, preferably between 85 and 200°C.

3. Process according to Claim 1 or 2, in which the operation is carried out at a pressure ranging from 5 to 30 bars, preferably between 10 and 20 bars.

4. Process according to one of Claims 1 to 3, in which 2 to 12 moles of $H_2S$, preferably from 4 to 8 moles, are employed per mole of di(tert-alkylthio)alkane.

5. Process according to one of Claims 1 to 4, in which the solid acidic catalyst is a natural or synthetic aluminosilicate or an ion exchange resin.

6. Process according to one of Claims 1 to 5, in which the di(tert-alkylthio)alkane is an α,ω-di(tert-alkylthio)alkane.

7. Process according to one of Claims 1 to 6, in which the tertiary mercaptan of formula :

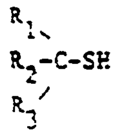

(II)

formed as a byproduct in the reaction is reemployed cyclically for the preparation of the starting di(tert-alkylthio)alkane.

8. Process according to Claim 7, in which the tertiary mercaptan (II) is chosen from those whose boiling point is sufficiently different from that of the final dithiol to enable them to be separated by simple distillation.

9. Process according to Claim 7 or 8, in which the tertiary mercaptan (II) is added photochemically to an unconjugated diene containing at least 5 carbon atoms.

10. Process according to Claim 9, in which from 2 to 4 moles of tertiary mercaptan are used per mole of diene.

11. Process according to Claim 9 or 10, in which the photochemical addition is performed at a temperature ranging from −10°C to +60°C, preferably between 20 and 50°C.

12. Process according to one of Claims 9 to 11, in which the photochemical addition is performed in the presence of a catalyst chosen from acetophenone derivatives, benzoylphosphine oxides and combinations of an aromatic ketone with an organic phosphite or phosphine.

13. Process according to one of Claims 9 to 12, in which the unconjugated diene is an α,ω-diene containing from 5 to 20 carbon atoms.

14. Process according to one of Claims 1 to 13, in which the operation is carried out continuously.

## Patentansprüche

1. Verfahren zur Herstellung von Dithiolen, dadurch gekennzeichnet, daß man in Gegenwart eines festen sauren Katalysators Schwefelwasserstoff mit einem Di-(tert.-alkylthio)-alkan der allgemeinen Formel

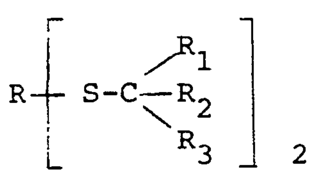

(I)

reagieren läßt, in der R einen 5 bis 20 Kohlenstoffatome enthaltenden Alkylenrest bedeutet und die Symbole $R_1$, $R_2$ und $R_3$ gleich oder verschieden sind und jeweils einen 1 bis 15 Kohlenstoffatome enthaltenden Alkylrest bedeuten.

2. Verfahren nach Anspruch 1, dadurch gekennzeichnet, daß die Umsetzung bei einer Temperatur zwischen Umgebungstemperatur und 250°C und vorzugsweise zwischen 85 und 200°C durchgeführt wird.

3. Verfahren nach Anspruch 1 oder 2, dadurch gekennzeichnet, daß man unter einem Druck von 5 bis 30 bar und vorzugsweise zwischen 10 und 20 bar arbeitet.

4. Verfahren nach einem der Ansprüche 1 bis 3, dadurch gekennzeichnet, daß man 2 bis 12 und vorzugsweise 4 bis 8 mol $H_2S$ pro mol Di-(tert.-alkylthio)-alkan umsetzt.

5. Verfahren nach einem der Ansprüche 1 bis 4, dadurch gekennzeichnet, daß der feste saure Katalysator ein natürliches oder synthetisches Aluminosilicat oder ein Ionenaustauscherharz ist.

6. Verfahren nach einem der Ansprüche 1 bis 5, dadurch gekennzeichnet, daß das Di-(tert.-alkylthio)-alkan ein α,ω-Di-(tert.-alkylthio)-alkan ist.

7. Verfahren nach einem der Ansprüche 1 bis 6, dadurch gekennzeichnet, daß das Reaktionsnebenprodukt, tertiäres Mercaptan der Formel

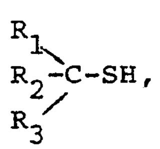

(II)

zur Herstellung des Ausgangsstoffs Di-(tert.-alkylthio)-alkan cyclisch wiederverwendet wird.

8. Verfahren nach Anspruch 7, dadurch gekennzeichnet, daß das tertiäre Mercaptan (II) aus solchen ausgewählt ist, deren Siedetemperatur von der des Dithiol-Endprodukts ausreichend verschieden ist, um ihre Trennung durch einfache Destillation zu erlauben.

9. Verfahren nach Anspruch 7 oder 8, dadurch gekennzeichnet, daß das tertiäre Mercaptan (II) photochemisch an ein wenigstens 5 Kohlenstoffatome enthaltendes nicht-konjugiertes Dien addiert wird.

10. Verfahren nach Anspruch 9, dadurch gekennzeichnet, daß man 2 bis 4 mol tertiäres Mercaptan pro mol Dien einsetzt.

11. Verfahren nach Anspruch 9 oder 10, dadurch gekennzeichnet, daß die photochemische Addition bei einer Temperatur von −10 bis +60°C und vorzugsweise zwischen 20 und 50°C durchgeführt wird.

12. Verfahren nach einem der Ansprüche 9 bis 11, dadurch gekennzeichnet, daß die photochemische Addition in Gegenwart eines Katalysators durchgeführt wird, der aus Acetophenon-Derivaten, Benzoyl- phosphinoxiden und den Verbindungen eines aromatischen Ketons mit einem organischen Phosphin oder Phosphit ausgewählt ist.

13. Verfahren nach einem der Ansprüche 9 bis 12, dadurch gekennzeichnet, daß das nicht-konjugierte Dien ein 5 bis 20 Kohlenstoffatome enthaltendes α,ω,-Dien ist.

14. Verfahren nach einem der Ansprüche 1 bis 13, dadurch gekennzeichnet, daß es kontinuierlich betrieben ist.